# FASCICULE DE BREVET EUROPEEN

(11) **EP 0 767 636 B1**
(45) Date de publication et mention de la délivrance du brevet: **20.01.1999**
(21) Numéro de dépôt: 95920938.8
(22) Date de dépôt: 11.05.1995
(51) Int. Cl.: A61F 2/44, A61F 2/46

(54) **IMPLANT VERTEBRAL**
WIRBELKÖRPERIMPLANTAT
VERTEBRAL IMPLANT

(30) Priorité: 11.05.1994 FR 9406028
(43) Date de publication de la demande: 16.04.1997
(73) Titulaire: Taylor, Jean, 06400 Cannes (FR)
(72) Inventeur: Taylor, Jean, 06400 Cannes (FR)
(74) Mandataire: Moncheny, Michel
(86) Numéro de dépôt international: FR9500623
(87) Numéro de publication internationale: WO9531158

(56) Documents cités:
- EP-A- 0 307 241
- WO-A-90/00037
- WO-A-92/14423
- DE-C- 4 012 622
- DE-U- 9 407 806
- US-A- 5 213 112

## Description

La présente invention concerne un implant vertébral et un instrument ancillaire pour la mise en place de cet implant.

La mobilité de deux vertèbres adjacentes se produit autour de trois points disposés en triangle, à savoir au niveau des deux apophyses articulaires postérieures, d'une part, et au niveau du disque inter-vertébral, d'autre part.

Les apophyses articulaires postérieures permettent les mouvements de flexion/extension et limitent les mouvements de rotation des vertèbres tandis que le disque, tout en ayant une fonction d'amortissement, permet les mouvements de rotation et limite les mouvements de flexion/extension.

L'asymétrie de l'espace discal, plus haut à sa partie antérieure que postérieure, participe à la lordose lombaire, en permettant à chaque disque de s'inscrire dans un arc de cercle à concavité postérieure. L'inclinaison moyenne du disque est appelée pente discale.

La défaillance de l'un de ces points d'articulation est à l'origine de phénomènes d'instabilité de la colonne vertébrale, conduisant à une dégénérescence et à un affaissement d'un ou plusieurs disques, notamment lombaires. La perte de cette asymétrie discale physiologique, qui aboutit à une insuffisance de lordose lombaire, est source d'un syndrome douloureux.

Le rétablissement d'une lordose lombaire se fait par voie antérieure ou postérieure, à l'aide, soit d'un greffon qui réalise une fusion, soit d'un matériel prothétique qui peut être implanté par voie transpéritonéale ou par voie postérieure.

Les prothèses implantées par voie postérieure sont cylindriques. Elles présentent deux inconvénients majeurs. D'une part, la forme de la prothèse fait que le contact est linéaire, et que l'implantation impose un avivement des surfaces sous-chondrales. D'autre part, leur forme supprime l'asymétrie discale en rétablissant simplement un certain degré de hauteur intersomatique, au prix d'une ankylose du segment antérieur vertébral.

La voie transpéritonéale est très iatrogène, tandis que la voie postérieure impose un important sacrifice osseux, avec un risque neurologique non négligeable.

La présente invention vise à remédier à ces différents inconvénients en fournissant un implant vertébral susceptible de restituer une lordose lombaire anatomique, sans affecter la mobilité inter-vertébrale et sans impliquer de geste chirurgical lourd et délicat.

Le document EP 0 307 241 décrit un implant monobloc de forme cylindrique ou parallélépipédique.

L'implant selon l'invention est du type apte à être inséré dans l'espace articulaire inter-vertébral par voie postérieure, et comprenant deux branches superposées, constituées par des éléments distincts, aptes chacune à venir en contact avec l'un des deux plateaux vertébraux, et comportant des moyens permettant de venir écarter ces branches l'une de l'autre.

Selon l'invention, l'implant est conformé pour présenter une hauteur postérieure restant constante et une hauteur antérieure variable, et en ce que les moyens permettant de venir écarter les branches sont conformés pour écarter les portions antérieures de ces deux branches sans écarter les portions postérieures de celles-ci.

Cet implant permet ainsi, sans intervention par voie antérieure, de rétablir l'asymétrie de la pente discale anatomique entre deux vertèbres adjacentes, notamment lombaires.

L'implant est introduit entre les vertèbres alors qu'il est en position de repos, les deux branches étant en position raprochée l'une de l'autre. Il a alors avantageusement une forme qui converge en direction de l'avant, la hauteur postérieure restant constante. Lors de leur écartement, les deux branches vont venir s'appuyer sur les plateaux adjacents à l'espace discal lorsqu'elles seront écartées l'une de l'autre. La hauteur antérieure de l'implant est ainsi variable et permet d'écarter les deux vertèbres.

A cet effet, la face postérieure de l'implant est avantageusement percée d'un trou taraudé, permettant l'introduction d'une vis, faisant alors acquérir à l'implant une divergence des deux branches reproduisant l'asymétrie de l'espace discal, plus ouvert en avant qu'en arrière. Au cours de cette distraction, les deux portions distales de l'implant, initialement au contact l'une de l'autre, s'éloignent, prennent contact avec le plateau vertébral, s'y appuient, permettant ensuite un effet de distraction asymétrique.

Suivant une forme de réalisation préférée de l'invention, simple à mettre en oeuvre, l'implant est constitué par pliage d'une lame métallique rendue élastique de par sa structure même, mais suffisamment solide grâce à son épaisseur et à un traitement thermique.

La partie centrale de la lame constitue la face postérieure de l'implant, et les parties latérales sont repliées sur elles-mêmes pour constituer les deux branches supérieures et inférieures.

Cette lame pourra être réalisée en alliage cuivre-béryllium, connu pour son élasticité.

On pourra préférer au cuivre-béryllium, le titane, pour les raisons suscitées, pour sa résistance et sa biocompatibilité.

Le principe d'élasticité variable de cet implant est apporté grâce à ce pliage particulier.

Un certain degré de rigidité pourra être apporté, si besoin est, par remplissage de chaque triangle à base supérieure et inférieure, que déterminent chacune des branches venant en appui contre les plateaux adjacents au disque ainsi instrumenté.

Cette souplesse élastique des deux branches de l'implant leur permet de ne pas constituer un obstacle, en deçà de leur limite élastique, aux mouvements de flexion/extension des vertèbres. Elles peuvent également présenter un certain degré de mobilité dans un plan sagittal, également de manière à ne pas constituer un obstacle aux mouvements de flexion/extension des vertèbres.

Avantageusement, les moyens d'écartement précités sont conformés pour écarter les deux branches de l'implant au-delà de ce qui est nécessaire pour rétablir l'asymétrie de la pente discale anatomique. Un tel écartement, en particulier lorsqu'il se combine avec la souplesse élastique desdites branches, permet de précontraindre l'implant pour l'obtention d'un maintien souple et dynamique des vertèbres en lordose.

Par ailleurs, les surfaces des deux branches de l'implant venant au contact avec l'os peuvent comprendre des aspérités venant s'insérer dans l'os, et/ou un traitement favorisant l'ostéo-intégration de l'implant, tel qu'un revêtement d'hydroxyapatite, une perforation ou un traitement chimique pour obtenir une oxydation du titane en surface.

Bien entendu, les implants peuvent avoir plusieurs tailles pour s'adapter aux patients.

L'instrument ancillaire permettant la mise en place de l'implant présente une extrémité reproduisant fidèlement l'ensemble de l'implant, des moyens pour l'insertion et l'extration de cette extrémité dans la cavité destinée à recevoir l'implant et des moyens permettant, à distance de cette extrémité, de venir écarter l'une de l'autre les portions antérieures des deux branches de cette extrémité sans écarter les portions postérieures de celles-ci. De préférence, l'instrument comprend des moyens de mesure de la force nécessaire à l'écartement de ces branches.

Cet instrument permet d'effectuer une préparation précise de la cavité de réception de l'implant, de préparer les zones de contact, de déterminer la taille de l'implant approprié et de faciliter ensuite la mise en tension de l'implant.

Pour sa bonne compréhension, l'invention est à nouveau décrite ci-dessous en référence au dessin schématique annexé représentant, à titre d'exemple non limitatif, une forme de réalisation préférée de l'implant vertébral qu'elle concerne.
La figure 1 en est une vue en perspective éclatée ;
la figure 2 en est une vue de côté, avant implantation ;
la figure 3 en est une vue de côté, après implantation, et
la figure 4 est une vue de profil d'un instrument ancillaire permettant la mise en place de l'implant.

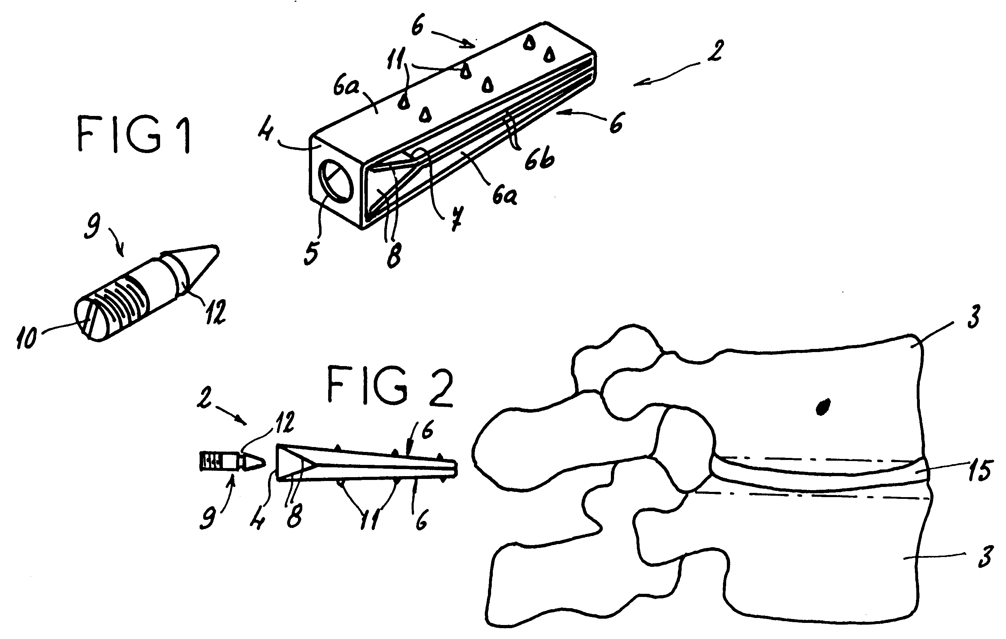

Les figures 1 à 3 représentent un implant vertébral 2 destiné à être inséré par voie postérieure entre deux vertèbres 3, notamment entre deux vertèbres lombaires.

Cet implant 2 est constitué par pliage d'une lame métallique souple et élastique, notamment en titane.

La partie centrale de la lame constitue la face postérieure 4 de l'implant 2. Cette face 4 est percée d'un trou taraudé 5.

Les deux parties latérales de la lame sont repliées à 180 degrés sur elles-mêmes puis repliées perpendiculairement à la face postérieure 4, pour former deux branches 6 superposées, parallèles entre elles et perpendiculaires à la face postérieure 4. Les branches 6 présentent chacune une portion extérieure 6a et une portion intérieure 6b. Les portions extérieures 6a sont sensiblement rectilignes tandis que les portions intérieures 6b sont pliées autour d'un axe 7 transversal à la lame de manière à définir, du côté de la face postérieure 4, deux portions arrière inclinées 8, situées en face du trou 5 et convergeant l'une vers l'autre dans la direction opposée à ce trou 5.

L'implant 2 comprend en outre une vis 9 apte à être engagée dans le trou 5 et à venir porter, au cours de son vissage à travers ce dernier, contre les deux portions arrière convergentes 8 précitées. L'extrémité de la vis 9 venant porter contre ces dernières est conique. Son autre extrémité comprend une empreinte diamétrale 10 permettant l'engagement d'un outil de manoeuvre en rotation. Dans la forme de réalisation représentée au dessin, la vis 9 n'est filetée que sur une portion de sa longueur. Cette vis comprend, en arrière de son extrémité conique, une gorge 12 permettant son maintien entre les branches 6 lorsque l'implant est mis en position d'ouverture, c'est-à-dire de distraction optimum.

Les portions extérieures 6a des branches 6 comprennent des aspérités 11 aptes à venir s'insérer dans l'os des plateaux vertébraux, ainsi qu'un traitement de surface, favorisant l'ostéo-intégration.

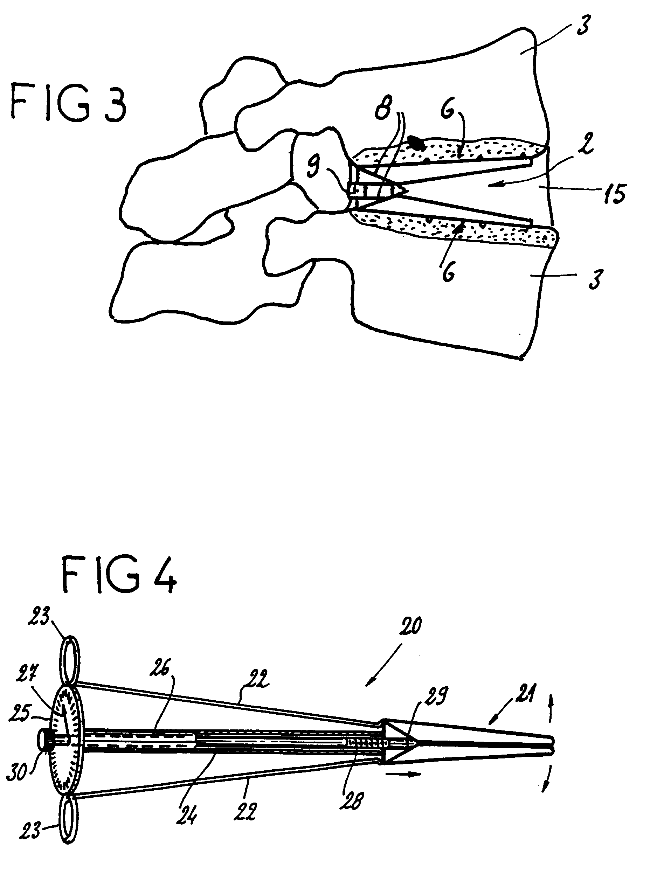

En pratique, une cavité adéquate est tout d'abord aménagée entre deux vertèbres 3 dont le disque 15 est défectueux. Comme le montre la figure 2, cette défectuosité entraîne une réduction importante de la hauteur discale avec, notamment, perte de l'asymétrie discale et avec des plateaux vertébraux qui, au lieu d'avoir une direction convergente vers l'arrière, deviennent parallèles.

La préparation de la cavité est effectuée à l'aide de l'instrument ancillaire 20 représenté à la figure 4.

Cet instrument 20 présente une extrémité 21 reproduisant fidèlement l'ensemble de l'implant 2, à laquelle deux prolongements 22 sont reliés. Ces prolongements 22 comportent des anneaux 23 à leur extrémité libre, assurant la préhension de l'instrument. Un tube 24 est fixé à la face arrière de l'extrémité 21, coaxialement au trou taraudé que celle-ci comprend. Il est muni à son autre extrémité d'un disque gradué 25 formant indicateur dynamométrique. Une tige 26, comportant une aiguille 27 calée sur elle, une portion filetée 28, une extrémité conique 29 et un profil 30 pour la prise d'appui d'une clef de manoeuvre en rotation, est engagée dans le tube 24 et dans le trou précité. Cette extrémité conique 29 vient en appui contre les portions arrière internes des branches de l'extrémité 21 lorsque la portion filetée 28 est en prise avec le taraudage du trou, tandis que l'aiguille 27 se trouve à proximité du disque 25.

Les tiges 22 permettent l'insertion et l'extraction de l'extrémité 21 dans la cavité destinée à recevoir l'implant 2. Le vissage de la tige 26 permet, à distance de cette extrémité 21, de venir écarter l'une de l'autre les portions antérieures des deux branches de cette extrémité 21 sans écarter les portions postérieures de celles-ci. L'aiguille 27, en référence à l'indication fournie par le dynamomètre 25, permet de mesurer le couple nécessaire à l'écartement de ces branches.

Cet instrument 20 permet d'effectuer une préparation précise de la cavité de réception de l'implant 2, de préparer les zones de contact, de déterminer la taille de l'implant 2 approprié et de faciliter ensuite la mise en tension de l'implant.

Après préparation adéquate, ce dernier est introduit dans la cavité.

Si besoin est, deux implants peuvent être insérés parallèlement dans le même espace inter-vertébral, de part et d'autre du sac dural.

Une fois l'implant convenablement mis en place, la vis 9 est engagée dans le trou 5. Au cours de son vissage, en venant porter contre les portions arrière internes des branches 6, elle permet d'écarter l'une de l'autre les portions antérieures des deux branches 6.

Le débattement des deux branches 6 étant supérieur à l'écartement inter-corporéal désiré, on assure ainsi une application dynamique en pression, en évitant une luxation de l'implant. Le degré du pliage interne de la lame métallique détermine ce degré d'ouverture. La vis 9 est immobilisée grâce au contact intime entre l'orifice 5 à la face postérieure de l'implant et le pas de vis, grâce à un système d'expansion (non représenté) qu'elle comprend avantageusement au niveau de sa tête et, enfin, par l'existence de la gorge 12, qui s'engage au niveau des arêtes séparant les portions arrière 8 des portions internes 6b des branches 6.

L'implant 2 permet ainsi de rétablir la pente discale anatomique, c'est-à-dire asymétrique, par voie postérieure (figure 3).

Grâce à sa constitution à partir d'une lame métallique souple et élastique, ses deux branches 6 présentent longitudinalement une certaine souplesse élastique ainsi qu'un certain degré de mobilité dans un plan horizontal. Elles ne constituent dès lors pas un obstacle, en deçà de leur limite élastique, aux mouvements de flexion/extension ou de rotation des vertèbres 3.

Le vissage est de préférence opéré de manière à ce que les deux branches 6 soient écartées au-delà de ce qui est nécessaire pour rétablir l'asymétrie de la pente discale anatomique. Cet écartement permet, en combinaison avec la souplesse élastique des branches 6, de précontraindre l'implant 2 pour obtenir un maintien souple et dynamique des vertèbres 3 en lordose.

S'il s'avère nécessaire de rigidifier les portions antérieures des branches 6, l'espace délimité par les portions extérieures 6a et intérieures 6b des branches 6 peut recevoir un matériau de renforcement venant le remplir totalement ou partiellement.

Il va de soi que l'invention n'est pas limitée à la forme de réalisation décrite ci-dessus à titre d'exemple mais qu'elle en embrasse au contraire toutes les variantes de réalisation.

Ainsi, la vis 9 pourrait être remplacée par une came excentrée pouvant être placée dans deux positions, à savoir une position inactive, dans laquelle les deux branches 6 sont en position rapprochée l'une de l'autre, et une position active, dans laquelle cette came écarte les deux branches 6.

L'implant 2 peut être réalisé en plusieurs éléments assemblés. Notamment, les branches 6 et la face postérieure 4 peuvent être constituées par des éléments distincts, articulés les uns aux autres.

En outre, la portion antérieure de l'implant pourrait prendre une forme ovalisée, de façon à mieux suivre les contours anatomiques de la portion antérieure des vertèbres, et éviter, lors de la distraction, un phénomène d'enfoncement dans les vertèbres. Pour la même raison, la portion distale de l'implant pourrait présenter une forme plus ou moins bombée.

La portion interne de l'implant, qui entre en contact avec la vis 9 ou avec la came, pourrait être décalée vers l'avant par rapport à l'implant représenté aux figures, de façon à réduire les contraintes de traction postérieures.

L'implant pourrait également comprendre deux parties emboîtées postérieurement, de façon à réduire d'éventuels phénomènes de vrillage.

## Revendications

1. Implant vertébral, apte à être inséré dans l'espace articulaire inter-vertébral par voie postérieure, et comprenant deux branches (6) superposées, constituées par des éléments distincts, aptes chacune à venir en contact avec l'un des deux plateaux vertébraux, et comportant des moyens (5,8,9) permettant de venir écarter ces branches (6) l'une de l'autre, implant caractérisé en ce qu'il est conformé pour présenter une hauteur postérieure restant constante et une hauteur antérieure variable, et en ce que les moyens (5,8,9) permettant de venir écarter les branches (6) sont conformés pour écarter les portions antérieures de ces deux branches (6) sans écarter les portions postérieures de celles-ci.

2. Implant vertébral selon la revendication 1, caractérisé en ce qu'il a une forme qui converge en direction de l'avant lorsque les deux branches (6) sont en position rapprochée l'une de l'autre.

3. Implant vertébral selon la revendication 1 ou la revendication 2, caractérisé en ce que les deux portions distales de l'implant sont initialement, c'est-à-dire avant l'écartement des branches (6), au contact l'une de l'autre.

4. Implant vertébral selon l'une des revendications 1 à 3, caractérisé en ce que les branches (6) présentent longitudinalement une certaine souplesse élastique, de manière à ne pas constituer un obstacle, en deçà de leur limite élastique, aux mouvements de flexion/extension des vertèbres (3).

5. Implant vertébral selon l'une des revendications 1 à 4, caractérisé en ce que ces deux branches (6) présentent un certain degré de mobilité dans un plan sagittal, de manière à ne pas constituer un obstacle aux mouvements de flexion/extension des vertèbres (3).

6. Implant vertébral selon l'une des revendications 1 à 5, caractérisé en ce que les moyens d'écartement (5,8,9) précités sont conformés pour écarter ses deux branches (6) au-delà de ce qui est nécessaire pour rétablir la pente discale anatomique, c'est-à-dire avec une asymétrie antéro-postérieure.

7. Implant vertébral selon l'une des revendications 1 à 6, caractérisé en ce que sa face postérieure (4) est percée d'un trou taraudé (5) ; en ce que les branches (6) précitées comprennent, aménagées dans leurs faces en regard, deux portions arrière inclinées (8) qui sont situées en face de ce trou (5) et qui convergent l'une vers l'autre dans la direction opposée à ce trou (5) ; et en ce qu'il comprend une vis (9) qui vient porter, lorsqu'elle est vissée au travers du trou taraudé (5), contre ces deux portions arrière convergentes (8) de manière à écarter les portions antérieures des deux branches (6) de l'implant.

8. Implant vertébral selon l'une des revendications 1 à 7, caractérisé en ce qu'il est constitué par pliage d'une lame métallique élastique, dont la partie centrale constitue sa face postérieure (4) et dont les parties latérales, repliées sur elles-mêmes, constituent les branches (6) précitées.

9. Implant vertébral selon l'une des revendications 1 à 7, caractérisé en ce que les surfaces de ses deux branches (6) venant au contact avec l'os comprennent des aspérités (11) venant s'insérer dans l'os, et/ou un traitement favorisant l'ostéo-intégration de l'implant, tel qu'un revêtement d'hydroxyapatite, une perforation ou un traitement chimique pour obtenir une oxydation du titane en surface.

10. Implant vertébral selon l'une des revendications 8 ou 9, caractérisé en ce que l'espace délimité par les portions extérieures (6a) et intérieures (6b) des branches (6) peut recevoir un matériau de renforcement pouvant remplir cet espace totalement ou partiellement.

11. Instrument ancillaire pour la mise en place d'un implant vertébral selon l'une des revendications 1 à 10, présentant une extrémité (21) reproduisant fidèlement l'ensemble de l'implant (2) et des moyens (22) pour l'insertion et l'extraction de cette extrémité (21) dans la cavité destinée à recevoir l'implant, cet instrument étant caractérisé en ce qu'il comprend des moyens (26 ; 28 à 30) permettant, à distance de cette extrémité (21), de venir écarter l'une de l'autre les portions antérieures des deux branches de cette extrémité (21) sans écarter les portions postérieures de celles-ci.

12. Instrument ancillaire selon la revendication 11, caractérisé en ce qu'il comprend des moyens (25,27) de mesure de la force nécessaire à l'écartement de ces branches.

## Claims

1. Vertebral implant which can be inserted into the inter-vertebral joint space from behind, and comprising two superposed branches (6) consisting of separate elements, each of which is able to come into contact with one of the two vertebral plates, and comprising means (5, 8, 9) allowing these branches (6) to be parted from one another, the implant being characterized in that it is shaped to have a posterior height that remains constant and an anterior height that can be varied, and in that the means (5, 8, 9) allowing the branches (6) to be parted are shaped to part the anterior portions of these two branches (6) without parting the posterior portions thereof.

2. Vertebral implant according to Claim 1, characterized in that it has a shape which converges towards the front when the two branches (6) are in the close-together position.

3. Vertebral implant according to Claim 1 or Claim 2, characterized in that the two distal portions of the implant are, initially, that is to say before the branches (6) are parted, in contact with one another

4. Vertebral implant according to one of Claims 1 to 3, characterized in that the branches (6) longitudinally have a certain elastic flexibility so that they do not, until they reach their limit of elasticity, impede the flexing/extending movements of the vertebrae (3).

5. Vertebral implant according to one of Claims 1 to 4, characterized in that these two branches (6) have a certain degree of mobility in a sagittal plane, so that they do not impede the flexing/extending movements of the vertebrae (3).

6. Vertebral implant according to one of Claims 1 to 5, characterized in that the aforementioned parting means (5, 8, 9) are shaped to part its two branches (6) by further than is required to re-establish the anatomical discal slope, that is to say with anterio-posterior asymmetry.

7. Vertebral implant according to one of Claims 1 to 6, characterized in that its posterior face (4) is pierced with a tapped hole (5); in that the aforementioned branches (6) comprise, formed in their opposing faces, two inclined rear portions (8) which are situated facing this hole (5) and which converge towards one another in the direction away from this hole (5); and in that it comprises a screw (9) which, when screwed through the tapped hole (5) bears against these two converging rear portions (8) so as to part the anterior portion, of the two branches (6) of the implant.

8. Vertebral implant according to one of Claims 1 to 7, characterized in that it is formed by folding an elastic metal leaf, of which the central part constitutes its posterior face (4), and of which the lateral parts, which are folded over on themselves, constitute the aforementioned branches (6).

9. Vertebral implant according to one of Claims 1 to 7, characterized in that those surfaces of its two branches (6) which come into contact with the bone have roughnesses (11) which bite into the bone, and/or comprise a treatment encouraging the osteo-integration of the implant, such as a hydroxyapatite coating, a perforation or a chemical treatment in order to obtain surface oxidation of the titanium.

10. Vertebral implant according to either of Claims 8 and 9, characterized in that the space delimited by the outer (6a) and inner (6b) portions of the branches (6)can accomodate a reinforcing material which may completely or partially fill this space.

11. Ancillary instrument for fitting a vertebral implant according to one of Claims 1 to 10, having one end (21) which faithfully reproduces the entire implant (2) and means (22) for inserting and extracting this end (21) in the cavity intended to receive the implant, this instrument being characterized in that it comprises means (26; 28 to 30) which allow the interior portions of the two branches of this and (21) to be parted from one another at some distance from this end (21)without parting the posterior portions of these branches.

12. Ancillary instrument according to Claim 11, characterized in that it comprises means (25, 27) for measuring the force needed to part these branches.

## Patentansprüche

1. Wirbelimplantat, das in den Zwischen wirbelgelenkraum auf dorsalem Weg eingesetzt werden kann und zwei übereinander angeordnete Schenkel (6), die aus getrennten Elementen bestehen und jeweils mit einem der beiden Wirbelkörperplateaus in Kontakt kommen können, sowie Einrichtungen (5, 8. 9) aufweist, die diese Schenkel (6) auseinanderspreizen können, dadurch gekennzeichnet, daß es so ausgebildet ist, daß es eine bleibende konstante hintere Höhe und eine veränderliche vordere Höhe besitzt, und daß die Einrichtungen (5, 8, 9), die die Spreizung der Schenkel (6) gestatten, so ausgebildet sind, daß die vorderen Bereiche dieser beiden Schenkel (6) gespreizt werden, ohne daß ihre hinteren Bereiche voneinander entfernt werden.

2. Wirbelimplantat nach Anspruch 1, dadurch gekennzeichnet. daß es eine nach vorne konvergierende Form hat, wenn die beiden Schenkel (6) in der einander angenäherten Stellung sind.

3. Wirbelimplantat nach Anspruch 1 oder 2, dadurch gekennzeichnet, daß die beiden distalen Bereiche des Implantats anfangs, d.h. vor dem Spreizen der Schenkel (6), miteinander in Kontakt sind.

4. Wirbelimplantat nach einem der Ansprüche 1 bis 3, dadurch gekennzeichnet, daß die Schenkel (6) in Längsrichtung eine gewisse elastische Biegsamkeit besitzen, so daß sie unterhalb ihrer Elastizitätsgrenze die Flexions/Extensions-Bewegungen der Wirbel (3) nicht behindern.

5. Wirbelimplantat nach einem der Ansprüche 1 bis 4, dadurch gekennzeichnet, daß die beiden Schenkel (6) in einer Sagittalebene einen gewissen Mobilitätegrad besitzen, so daß sie die Flexions/Extensions-Bewegungen der Wirbel (3) nicht behindern.

6. Wirbelimplantat nach einem der Ansprüche 1 bis 5, dadurch gekennzeichnet, daß die Spreizeinrichtungen (5, 8, 9) so ausgebildet sind, daß aie ihre beiden Schenkel (6) über das hinaus spreizen, was erforderlich ist, um die anatomische Diskusneigung, d.h. Mit einer doreal-ventralen Asymmetrie, wieder herzustellen.

7. Wirbelimplantat nach einem der Ansprüche 1 bis 6, dadurch gekennzeichnet, daß ihre hintere Seite (4) mit einer Gewindebohrung (5) versehen ist, daß die Schenkel (6) auf ihren einander gegenüberstehenden Seiten zwei hintere, schräge Teile (8) aufweisen, die dieser Bohrung (5) gegenüberliegen und in der dieser Bohrung entgegengesetzten Richtung zueinander konvergieren, und däß es eine Schraube (9) aufweist, die an diesen beiden konvergierenden hinteren Teilen (8) zum Anliegen kommt, wenn sie durch die Gewindebohrung (5) geschraubt ist, so daß die vorderen Bereiche der beiden Schenkel (6) des Implantats gespreizt werden.

8. Wirbelimplantat nach einem der Ansprüche 1 bis 7, dadurch gekennzeichnet, daß es durch Biegen einer elastischen Metallamelle gebildet ist, deren mittlerer Teil seine hintere Seite (4) und deren auf sich selbst umgebogenen seitlichen Teile die Schenkel (6) bilden.

9. Wirbelimplantat nach einem der Ansprüche 1 bis 7, dadurch gekennzeichnet, daß die Flächen seiner beiden Schenkel (6), die mit dem Knochen in Kontakt kommen, in den Knochen eintretende Unebenheiten (11) und/oder eine die Osteointegration des Implantats begünstigende Behandlung aufweisen, wie eine Hydroxyapatitbeschichtung, eins Perforierung oder eine chemische Behandlung, um eine Oxidation des Titans an der Oberfläche zu erhalten.

10. Wirbelimplantat nach einem der Ansprüche 8 oder 9, dadurch gekennzeichnet, daß der von den äußeren Teilen (6a) und den inneren Teilen (6b) der Schenkel (6) abgegrenzte Raum ein Verstärkungematerial aufnehmen kann, das diesen Raum vollständig oder teilweise füllen kann.

11. Hilfsinstrument zum Einsetzen eines Wirbelimplantats nach einem der Ansprüche 1 bis 10, das ein Ende (21) aufweist, das dem gesamten Implantat (2) getreu nachgebildet ist, sowie Einrichtungen (22) zum Einführen dieses Endes (21) in den zur Aufnahme des Implantats bestimmten Hohlraum und zur Entnahme dieses Endes aus diesem Hohlraum, dadurch gekennzeichnet, daß es Einrichtungen (26; 28 bis 30) besitzt, die es gestatten, von einer Entfernung von diesem Ende (21) aus die beiden Schenkel dieses Endes (21) in ihren vorderen Bereichen zu spreizen, ohne ihre hinteren Bereiche voneinander zu entfernen.

12. Hilfsinstrument nach Anspruch 11, dadurch gekennzeichnet, daß es Einrichtungen (25, 27) zum Messen der zum Spreizen dieser Schenkel erforderlichen Kraft aufweist.
